# EUROPEAN PATENT APPLICATION

(11) **EP 4 657 105 A1**
(43) Date of publication of application: **03.12.2025**
(21) Application number: 24179205.0
(22) Date of filing: 31.05.2024
(51) Int. Cl.: G01R 33/48, G01R 33/56, G01R 33/561, G01R 33/563, A61N 5/10, G01R 33/485, G01R 33/50, G01R 33/567

(54) **TURBO FIELD ECHO MAGNETIC RESONANCE IMAGING WITH AN INITIAL SPIN ECHO PREPARATORY PULSE**

(71) Applicant: Koninklijke Philips N.V., 5656 AG Eindhoven (NL)
(72) Inventor: BECK, Gabriele, Eindhoven (NL); KRUISKAMP, Marinus Johan, Eindhoven (NL); ALBERTS, Eveline, 5656AG Eindhoven (NL)
(74) Representative: Philips Intellectual Property & Standards

(57) **Abstract**

Disclosed herein is a medical system (100) comprising a magnetic resonance imaging system (102). Execution of the machine executable instructions (140) causes a computational system (132) to: repeatedly (200) acquire k-space data (144) in multiple shots (400) by controlling the magnetic resonance imaging system with pulse sequence commands (142) and reconstruct (202) at least one magnetic resonance image (146). The pulse sequence commands cause the magnetic resonance imaging system to perform an initial spin echo preparatory pulse (308) with a refocusing flip angle of 180° or less. The initial spin echo preparatory pulse prepares T2 or diffusion magnetization for each of the multiple shots, acquires the k-space data using a Turbo Field Echo readout (310) with T1 weighting, balanced or T2 weighting, and acquires the k-space data using a flip angle that decreases as a distance from a central k-space region increases.

## Description

### FIELD OF THE INVENTION

The invention relates to magnetic resonance imaging, in particular to the application of Turbo Field Echo readouts during magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. A combination of radio-frequency signals and magnetic gradients are used to encode the nuclear spins. The nuclear spins will emit radio- frequency signal in response to this encoding. These radio-frequency signals may be sampled and stored digitally. These samples are in samples in k-space and are referred to herein as k-space data. The k-space data may be Fourier transformed into a magnetic resonance image. A (TFE) Turbo field echo pulse sequence is a gradient or field echo pulse sequence that samples the k-space data in a shot after an initial preparation pulse for contrast enhancement.

### SUMMARY OF THE INVENTION

The invention provides for a medical system, a method, and a computer program in the independent claims. Embodiments are given in the dependent claims.

In one aspect a medical system is disclosed. The medical system comprises a magnetic resonance imaging system configured for acquiring k-space data from a subject at least partially within an imaging zone. The medical system further comprises a memory storing machine-executable instructions and pulse sequence commands configured to acquire the k-space data as multiple shots of k-space data. The pulse sequence commands are configured to cause the magnetic resonance imaging system to perform an initial spin echo preparatory pulse with a refocusing flip angle of 180° or less. The initial spin echo preparatory pulse is configured to prepare T2 or diffusion magnetization for each of the multiple shots. The pulse sequence commands are further configured to acquire the k-space data using a turbo field echo readout with T1, balanced or T2 weighting. The pulse sequence commands are configured to acquire the k-space data using a flip angle that decreases as the distance from a central k-space region increases.

The medical system further comprises a computational system. Execution of the machine-executable instructions causes the computational system to repeatedly acquire the k-space data in multiple shots by controlling the magnetic resonance imaging system with the pulse sequence commands. Execution of the machine-executable instructions further causes the computational system to reconstruct at least one magnetic resonance image from the multiple shots of k-space data according to the magnetic resonance imaging protocol.

In another aspect, a method of magnetic resonance imaging is disclosed. The method comprises repeatedly acquiring k-space data in multiple shots by controlling a magnetic resonance imaging system with pulse sequence commands. The pulse sequence commands are configured to cause the magnetic resonance imaging system to perform an initial spin echo preparatory pulse with a refocusing flip angle of 180° or less. The initial spin echo preparatory pulse is configured to prepare T2 or diffusion magnetization for each of the multiple shots. The pulse sequence commands are further configured to acquire the k-space data using a turbo field echo readout with T1, balanced weighting or T2 weighting. The pulse sequence commands are further configured to acquire the k-space data using a flip angle that decreases as a distance from a central k-space region increases. The method further comprises reconstructing at least one magnetic resonance image from the multiple shots of k-space data according to the magnetic resonance imaging protocol.

In another aspect, a computer program comprising machine-executable instructions is disclosed. The machine-executable instructions are configured for causing the computational system to perform the above method.

According to an aspect, there is provided a computer-readable medium having stored thereon the computer program as mentioned above. The medium may be non-transitory. For example, the medium may be a floppy disk, a CD, a DVD, or a solid-state memory device.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 shows a flow chart which illustrates a portion of a pulse sequence that incorporates T1 measurements after performing T2 measurements.
Fig. 4 illustrates a further example of a pulse sequence 400 for a single TFE shot.
Fig. 5 shows three different slices of a magnetic resonance image that compares three different imaging protocols.
Fig. 6 shows three different slices of a magnetic resonance image that also compares three different imaging protocols.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example the medical system comprises a magnetic resonance imaging system that is configured for acquiring k-space data from a subject at least partially within an imaging zone. Magnetic resonance imaging systems have magnets which generate large magnetic fields which is often referred to as the main magnetic field or the B0 field. The k-space data could be acquired using two-dimensional or three-dimensional or four-dimensional (dynamic or respiratory resolved) or five-dimensional (including cardiac) MRI imaging protocols. The imaging zone is a region where the magnetic field is high enough and uniform enough to enable magnetic resonance imaging. The medical system further comprises a memory storing machine-executable instructions and pulse sequence commands configured to acquire the k-space data as multiple shots of k-space data. A shot of k-space data as used herein encompasses k-space data that occurs during one partition of the pulse sequence.

The pulse sequence commands are configured to cause the magnetic resonance imaging system to perform an initial echo preparatory pulse with a refocusing flip angle of 180° or less. The initial spin echo preparatory pulse is configured to prepare T2 magnetization or diffusion magnetization. The preparatory pulse may consist of a preparatory excitation with a flip angle of 90° or less and refocusing pulse with a flip angle of 180° or less that can be either used standalone or by applying a reversed preparatory pulse scheme flipping the magnetization back to the longitudinal magnetization. The pulse sequence commands are further configured to acquire the k-space data using a turbo field echo readout with T1, balanced or T2 weighing. The pulse sequence commands are configured to acquire the k-space data using a flip angle that decreases as the distance from a central k-space region increases. A higher flip angle at the central k-space region and the use of the decreasing flip angles may be beneficial to increase the signal to noise ratio (SNR) and contrast to noise ratio (CNR) improving the TFE steady state behavior after the initial spin echo preparation but also reducing sequence timings due to a reduced SAR (specific absorption rate).

The medical system further comprises a computational system. Execution of the machine-executable instructions causes the computational system to repeatedly acquire the k-space data in the multiple shots by controlling the magnetic resonance imaging system with the pulse sequence commands. Execution of the machine-executable instructions further causes the computational system to reconstruct at least one magnetic resonance image from the multiple shots of k-space data according to the magnetic resonance imaging protocol.

The combination of the initial spin echo preparatory pulse with the T2 or diffusion magnetization in combination with the turbo field echo readout with T1, balanced or T2 weighting may be beneficial because it may provide for improved visualization and characterization of anatomical structures such as tumors, tumor bed, cysts whilst pressing background tissue efficiently. This may for example enable not only improved magnetic resonance images but other techniques which may use these magnetic resonance images. For example, it may provide for improved biological relevant synthetic CT images as well as the guidance of radiotherapy systems.

In another example, the pulse sequence commands are configured such that the k-space data is acquired using at least dual echo gradient echo readouts. At least a dual echo gradient echo readouts encompasses herein either using dual gradient echo readouts, for a 2-point Dixon technique, or using more than two gradient echo readouts for a multi-point Dixon technique. The at least one magnetic resonance image comprises a Dixon water image and/or a Dixon in-phase image and/or a Dixon fat image and/or a Dixon out-phase image and/or Dixon B0 image and/or Dixon T2* image and/or Dixon fat fraction image. The use of Dixon imaging enables the separation of the image not only into fat and water components but also B0, T2* and fat fraction components. This may provide for improved visualization of anatomical structures within the resulting magnetic resonance images providing an improved visualization of the tumor and tumor bed as well as an improved capacity to use these images to construct other synthetic images such as a distortion free synthetic CT images and/or to control other devices such as a radiotherapy device. However, it can be also used for treatment response monitoring following up on quantitative T2* and fat fraction values as an example. The use of the T2* and fat fraction values could be used for follow-up after the use of the radio therapy device as these two maps provide quantitative value.

In another example, execution of the machine-executable instructions further causes the computational system to reconstruct a synthetic computed tomography image using the Dixon water image and the Dixon fat image and the Dixon B0 map image. The use of the Dixon water image and the Dixon fat image may enable accurate prediction of the Hounsfield value for individual voxels within the synthetic computed tomography image. A model segmenting for bone tissue as well as approximating the percentage of water or fat within a voxel may be used by an algorithm to accurately predict the Hounsfield unit for each voxel. In other examples, the Dixon water image and the Dixon fat image and the Dixon B0 map image could be inserted into a neural network trained to predict the distortion free computed tomography image. This for example could be done with a U-Net or a GAN (cycle-GAN) network that is configured to receive the Dixon water image and the Dixon fat image and the Dixon B0 map image as input as an example and to output in response a single image which is the distortion free synthetic computed tomography image. The neural network could be trained either by acquiring and matching the Dixon water image and the Dixon fat image to a real computed tomography image or a synthetic computed tomography image may be calculated using an algorithmic approach and this may be used for synthetically creating the training data. By separately providing the relevant MR specific imaging characteristics to the neural network the synthetic computed tomography image may be improved not only with respect to the Hounsfield unit precision, the B0 field uncertainties and respective spectral and geometric accuracies but also with respect to tumor and tumor bed characteristics. The T2 prepared fat, T2* and fat fraction representation may provide relevant quantitative contrast information relevant for tumor and tumor bed characteristics. Fat content, oxygenation levels important for resistance of tumors to radiation showing hypoxic tumors may be classified. Also, synthetic contrast may be provided with a good CNR of the tumor.

In another example, execution of the machine-executable instructions further causes the computational system to receive a radiation therapy treatment plan identifying one or more regions of the subject to irradiate. Execution of the machine-executable instructions further causes the computational system to generate radiation therapy control commands configured to control a radiation therapy system to irradiate the one or more regions of the subject using the synthetic computed tomography image. Synthetic computed tomography images may provide for an accurate means of estimating the penetration of ionizing radiation as well as its absorption by different tissues within the subject. Using the synthetic computed tomography image the radiation therapy control commands may be generated more accurately.

In some instances, the synthetic computed tomography image may be four- or five dimensional. For example, it may be time dependent and may be arranged such that it is dependent upon a breathing or respiratory phase of the subject. In this case the radiation therapy treatment plan may be able to generate respiratory-dependent radiation therapy control commands for controlling the radiation therapy system.

In another example, the pulse sequence commands are configured to acquire the multiple shots of k-space data with a golden angle sampling pattern that is varied by shot and by pulse sequence repetition. This may be beneficial because it may provide for an effective means of distributing the k-space samples such that they can be binned and used for making four-dimensional or respiratory-resolved magnetic resonance images.

In another example, the pulse sequence commands are configured such that the multiple shots of k-space data are acquired using interleaved phase cycling and / or correction (like sum of squares) of the different phase cycles for the balanced weighting. The advantage of using phase cycling may be that the artifacts in the image may for example be reduced by the change of phase errors and/or artifacts that may occur due to imperfect radio frequency pulses or magnetic field inhomogeneity. The use of the phase cycling may provide for an optimal banding artifact level in the resulting magnetic resonance image.

In another example, the medical system further comprises a respiratory monitoring system configured for measuring respiratory data descriptive of a respiratory cycle of the subject. The respiratory monitoring system could, for example, be any one of the following: an external k-space navigator, an intrinsic (self) k-space navigator, a FID navigator, an external sensor (3D infrastructural light camera), and combinations thereof. Execution of the machine executable instructions causes the computational system to repeatedly: acquire the multiple shots of k-space data by controlling the magnetic resonance imaging system with the pulse sequence commands, acquire the respiratory data using the respiratory monitoring system during the acquisition of the multiple shots of k-space data, and label the multiple shots of the k-space data using the respiratory data based on a time matching the respiratory data. Execution of the machine executable instructions further causes the computational system to bin the labeled multiple shots of the k-space data by the respiratory cycle. The at least one magnetic resonance image is multiple magnetic resonance images for the respiratory cycle. In another example, the medical system further comprises a respiratory monitoring system configured for measuring respiratory data descriptive of a respiratory cycle and time aligned of the subject. Execution of the machine-executable instructions causes the computational system to repeatedly acquire the multiple shots of k-space data by controlling the magnetic resonance imaging system with the pulse sequence commands such that the multiple shots occur at varying delays after the initial spin echo preparatory pulse. Execution of the machine-executable instructions further causes the computational system to acquire the respiratory data using the respiratory monitoring system during the acquisition of the multiple shots of k-space data.

Execution of the machine-executable instructions further causes the computational system to label the multiple shots of the k-space data using the respiratory data based on a time-matching of the varying delays and the respiratory data. Execution of the machine-executable instructions further causes the computational system to bin the labeled multiple shots of the k-space data by the respiratory cycle and by the varying delays. The at least one magnetic resonance image may be multiple magnetic resonance images which comprise images corresponding to different phases of the respiratory cycle and for the respective varying delays.

In another example, the pulse sequence commands are configured such that the turbo field echo readout has a T1, balanced or T2 weighting. Execution of the machine-executable instructions further causes the computational system to calculate a time-dependent T2 relaxation time map from the at least one magnetic resonance image for the respiratory phase and for the varying delays. This example may be beneficial because it may provide for an improved T2 relaxation time map that is resolved for the respiratory phase of the subject.

In another example, the pulse sequence commands are configured to cause the magnetic resonance imaging system to perform an inversion pulse after acquisition of the k-space data. The pulse sequence commands are further configured to acquire T1 k-space data after the inversion pulse. Execution of the machine-executable instructions causes the computational system to repeatedly acquire the T1 k-space data in multiple shots by controlling the magnetic resonance imaging system with the pulse sequence commands. The multiple shots occur at varying time after the inversion pulse. Execution of the machine-executable instructions further causes the computational system to repeatedly acquire the respiratory data using the respiratory monitoring system during the acquisition of the T1 k-space data. Execution of the machine-executable instructions further causes the computational system to repeatedly label the multiple shots of the T1 k-space data using the multiple respiratory data based on a time-matching of the varying delays and the respiratory data.

Execution of the machine-executable instructions further causes the computational system to bin the labeled multiple shots of the T1 k-space data by the respiratory data and by the varying times after the inversion pulse. Execution of the machine-executable instructions further causes the computational system to reconstruct a T1-weighted image for the respiratory phase and for the respective varying times. Execution of the machine-executable instructions further causes the computational system to calculate a four-dimensional T1 relaxation time map from the T1-weighted image for the respiratory phase and for the varying times.

In another example, execution of the machine-executable instructions further causes the computational system to generate a four-dimensional synthetic magnetic resonance image from the four-dimensional T2 relaxation time map and the four-dimensional T1 relaxation time map.

In another example, execution of the machine-executable instructions further causes the computational system to segment the four-dimensional synthetic magnetic resonance image using a segmentation algorithm to identify anomalous anatomical structures. Execution of the machine-executable instructions further causes the computational system to overlay the segmentation of the anomalous anatomical structures on the four-dimensional synthetic magnetic resonance image. This example may be beneficial because it may provide for an improved means of segmenting the magnetic resonance images. The segmentation algorithm may for example be implemented in different ways. An edge detection algorithm, which detects the edges or abrupt changes in the contrast of the magnetic resonance image may be used. In other examples, more sophisticated AI based segmentation algorithms may be used that incorporate synthetic creation of contrasts from the quantitative T1, T2, T2* and FF that optimizes the tumor and tumor bed characterization with respect to biological and treatment relevant information such as oxygenation based on T1 and T2* or diffusion or fat fraction. In other examples, a deformable shape model may be fit to the four-dimensional synthetic magnetic resonance image. For example, for each time or respiratory phase the implementation may be performed for each of these individual images.

In another example, startup cycles and intrinsic external k-space navigators for the respiratory monitoring system is integrated into the pulse sequence commands. Execution of the machine-executable instructions further causes the computational system to switch the additional external k-space navigators between being leading navigators to trailing navigators and removing any startup cycles. This example may be beneficial because it may provide for a means of improving the T2 or diffusion contrast efficiency and / or navigator robustness.

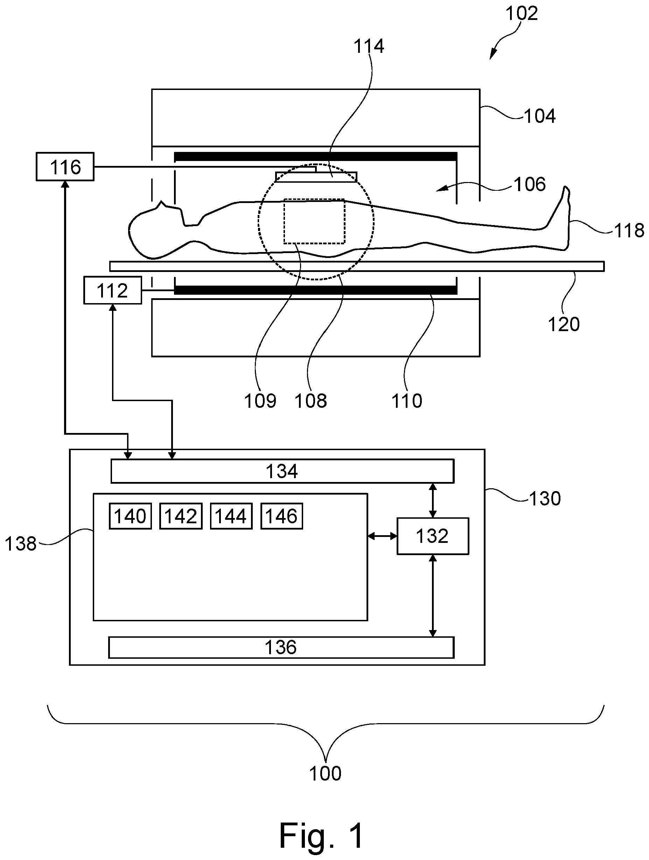

Fig. 1 illustrates an example of a medical system 100 that comprises a magnetic resonance imaging system 102 and a computer 130. The magnetic resonance imaging system 102 comprises a magnet 104. The magnet 104 is a superconducting cylindrical type magnet with a bore 106 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 106 of the cylindrical magnet 104 there is an imaging zone 108 where the magnetic field is strong and uniform enough to perform magnetic resonance imaging. A field of view 109 is shown within the imaging zone 108. The k-space data are acquired for the field of view 109. The region of interest could be identical with the field of view 109 or it could be a sub volume of the field of view 109. A subject 118 is shown as being supported by a subject support 120 such that at least a portion of the subject 118 is within the imaging zone 108 and the field of view 109.

Within the bore 106 of the magnet there is also a set of magnetic field gradient coils 110 which is used for the acquisition of measured k-space data to spatially encode magnetic spins within the imaging zone 108 of the magnet 104. The magnetic field gradient coils 110 are connected to a magnetic field gradient coil power supply 112. The magnetic field gradient coils 110 are intended to be representative. Typically, magnetic field gradient coils 110 contain three separate sets of coils for spatial encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio frequency coil 114 is connected to a radio frequency transceiver 116. The radio frequency coil 114 and radio frequency transceiver 116 may be replaced by separate transmit and receive coils and a separate transmitter and receiver. It is understood that the radio frequency coil 114 and the radio frequency transceiver 116 are representative. The radio frequency coil 114 is intended to also represent a dedicated transmit antenna and a dedicated receive antenna. Likewise, the transceiver 116 may also represent a separate transmitter and receiver. The radio frequency coil 114 may also have multiple receive/transmit elements and the radio frequency transceiver 116 may have multiple receive/transmit channels. The transceiver 116 and the gradient controller 112 are shown as being connected to the hardware interface 106 of the computer system 102.

The transceiver 116, the magnetic field gradient coil power supply 112 and the EMI detector 122 are shown as being connected to a hardware interface of computer 130. The computer 130 is further shown as comprising a computational system 132. The computer 130 and the computational system 132 may be one or more computer systems or computational systems located at one or more locations. The hardware interface 134 may enable the computational system 132 to communicate with and to control other components of the medical system 100. The computational system 132 is further shown as being in communication with an optional user interface 136 and a memory 138. The memory 138 is intended to represent different types of memory which may be accessible to the computational system 132.

The memory 138 is shown as containing machine-executable instructions 140. The machine-executable instructions 140 enable the computational system 132 to perform such tasks as controlling the magnetic resonance imaging system 102 and performing numerical and image processing tasks. The memory 138 is further shown as containing pulse sequence commands 142.

The pulse sequence commands 142 are configured to cause the magnetic resonance imaging system to perform an initial spin echo preparatory pulse with a refocusing flip angle of 180 degree or less, wherein the initial spin echo preparatory pulse is configured to prepare T2 or diffusion magnetization for each of the multiple shots. The pulse sequence commands are further configured to acquire the k-space data using a Turbo Field Echo readout with a T1, balanced or T2 weighting. The pulse sequence commands are configured to acquire the k-space data using a flip angle that decreases as a distance from a central k-space region increases.

The memory 138 is further shown as containing k-space data 144 that was acquired by controlling the magnetic resonance imaging system 102 with the pulse sequence commands 142. The k-space data 144 is divided into different groups or shots of k-space data. The memory 138 is further shown as containing a magnetic resonance image 146 that was reconstructed from the k-space data 144.

The medical system 100 illustrated in Fig. 1 could also comprise a system for measuring a respiratory phase of the subject. This could be used to bin the k-space data by the respiratory phase of the subject and used to construct time dependent magnetic resonance images. For example, if the shots of k-space data are acquired using a three-dimensional protocol then the system for measuring the respiratory phase could be used to construct four-dimensional magnetic resonance images (three-dimensional with a time dependence).

The system of measuring a respiratory phase could be implemented in different ways. For example, a camera could be used to monitor the respiratory phase. Other techniques such as the use of k-space navigators could also be used to monitor the respiratory phase.

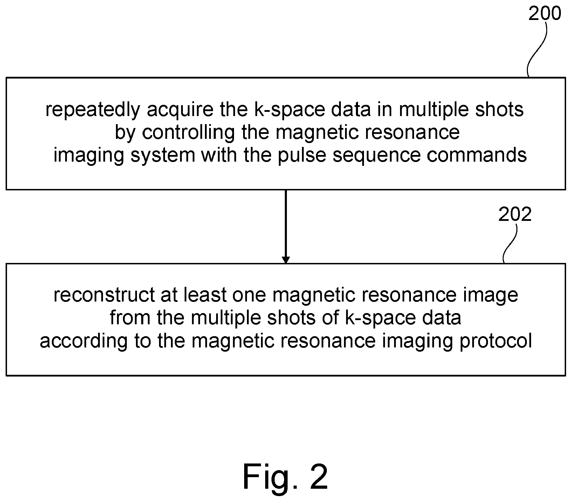

Fig. 2 shows a flowchart which illustrates a method of operating the magnetic resonance imaging system 102 of Fig. 1. In step 200 the k-space data 144 is repeatedly acquired in multiple shots by controlling the magnetic resonance imaging system 102 with the pulse sequence commands 142. In step 202 at least one magnetic resonance image 146 is reconstructed from the multiple shots of k-space data 144 according to the magnetic resonance imaging protocol.

The use of four-dimensional respiratory binning (three-dimensional plus time or respiratory phase) offers the possibility to track breathing motion for radiation therapy planning and motion management.

Despite the benefits, current four-dimensional technology does not provide the contrast needed for the depiction of tumorous tissue. Also, it does not allow to quantify and optimize the contrast. This, however is important for contouring, image registration and treatment planning where the identification of the tumor and the organs at risk over breathing states may be beneficial.

Examples may provide for an improved contrast depiction, quantification and synthetic contrast generation integrated within contouring and four-dimensional respiratory binning and synthetic four-dimensional Computer Tomography (CT) workflow. These concepts are generic with respect to 4D binning techniques and can be applied to radial, spiral stack of stars or cartesian CASPR techniques where the central k-space center is oversampled compared to the peripheral k-space.

Examples may comprise one or more of the following features:
- Using a T2 preparation module and a 3D gradient echo shot readout followed by an (partial) inversion or saturation pulse and one or more 3D gradient echo readouts (shots)
- Continuous golden angle distributed sampling using a central k-space oversampled acquisition method like 3D radial stack of stars, spiral stack of stars or 3D cartesian spiral (CASPR)
- with a different golden angle sampling per shot and source contrasts
- share subsequent profiles across shot acquisitions and source contrasts
- Detect breathing motion by (intrinsic, external added or FID) k-space navigator, pencil beam navigator or external sensors
- Bin the acquired k-space data with respect to the detected (breathing) motion and sampled contrast
- Reconstruct from the binned data 3D image data sets with different source contrasts and motion states using reconstruction methods like k-space weighting, KWIC filtering, compressed sense or artificial intelligence (AI)
- Create from the source contrast data sets, quantitative and synthetic MR data sets for improved contouring
- Acquire dual or multiple gradient echoes and use Dixon reconstruction to separate at least water and fat. From the separate water and fat reconstructions create a synthetic 4D CT for radiation therapy planning

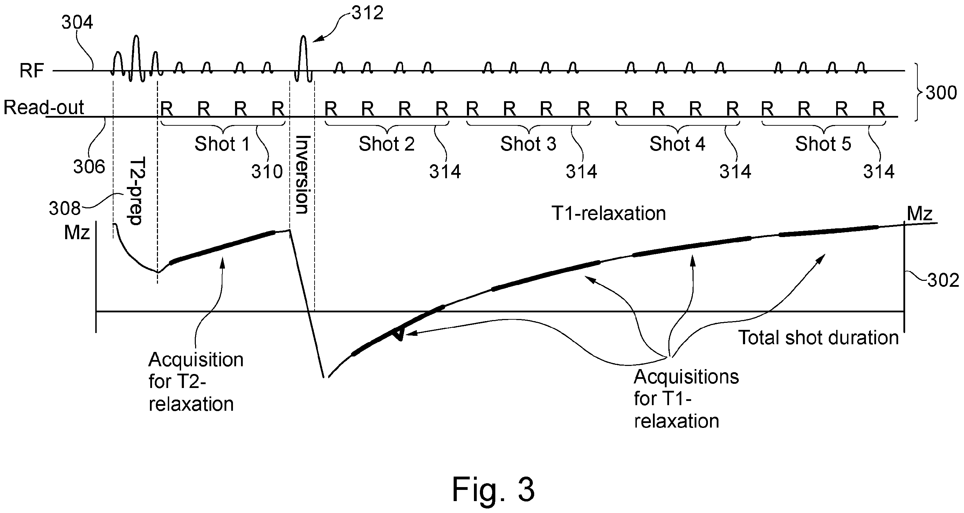

Fig. 3 shows a figure which shows a portion of a pulse sequence 300 which may be used for two-dimensional imaging, three-dimensional imaging, or may be used for four-dimensional respiratory binning as was discussed above. The RF pulses 304 and the readout portions 306 are illustrated. The portion of the pulse sequence 300 would acquire one shot of k-space data. After a T2 preparatory pulse 308 (T2 preparation module), there is a (two- or three-dimensional) gradient echo shot readout that may be a turbo field echo readout 310 where T2 relaxation k-space is measured. In this example, the T2 preparatory pulse is an example of an initial spin echo preparatory pulse. The T2 preparatory pulse may also be replaced with a diffusion magnetization preparatory pulse. In another one example, the pulse sequence may consist of only this first part (310) of the pulse sequence diagram with the T2 preparatory pulse 308 and a shot readout 306. Another pulse sequence example may consist of both the T2 prep and (partial) inversion or saturation pulse 312 and then a number of T1 k-space data acquisitions 314. One shot or pulse repetition acquires both T2 data and T1 data.

Continuous golden angle distributed sampling using a central k-space oversampled sampling method like 3D radial stack of stars, spiral stack of stars or 3D cartesian spiral (CASPR) may be used. In this example subsequent shots and contrasts use different golden angles. The use of different golden angles allows sharing subsequent profiles across shot acquisitions and contrasts. Also it allows synthesizing motion and source contrast information from a single acquisition making use of the redundant information between contrasts and motion.

Respiratory states may be detected by intrinsic k-space navigator, external sensors like the camera or pencil beam navigator.

The data may be respiratory and source contrast binned distinguishing inspiration and expiration states with rejection of outliers.

From the binned data, 3D image data sets with different source contrasts and motion states may be reconstructed incorporating reconstruction methods like k-space weighting, KWIC filtering, weighting within compressed sensing and artificial intelligence (AI).

From the respiratory resolved source contrast data sets, quantitative MR data may be synthesized. Another option is to create synthetic contrasts that improves the contrast of tumorous tissue improving auto contouring.

For radiation therapy planning synthetic CT data may be created. This can be achieved by incorporating a dual or multiple gradient echo acquisition and using a Dixon reconstruction to separate water and fat. From the separate water and fat image data and/or B0 map sets, synthetic 4D CT data sets can be synthesized for radiation therapy planning. In addition, a T2* relaxation time map, a four-dimensional fat fraction map, and/or a four-dimensional B0 map could, if available, also be used.

The pulse sequence 300 illustrated in Fig. 3 may be used, for example, in the context of four-dimensional respiratory resolved, motion robust, synthetic contrast and quantitative MR imaging allowing improved tumor visualization and classification based on quantitative MR within reasonable scan time.

Further examples may provide for an improved contrast depiction integrated in the four-dimensional respiratory binning and synthetic four-dimensional CT workflow. Note these concepts are generic with respect to 4D binning techniques and can be applied to radial, spiral stack of stars or cartesian CASPR techniques where the central k-space center is oversampled compared to the peripheral k-space.

Further examples, may have one or more of the following features:
- Using a T2 preparation module with a 3D gradient echo, preferably T1, T2 - shifted echo - or balanced gradient echo readout
- Optional include inversion pulse or a Magnetization Transfer Contrast (MTC) pulse to improve suppression of background (liver) signal
- Improve scanning efficiency by acquiring immediately after the inversion pulse with placement of the central k-space acquisition at the most optimal inversion delay time continued with the skipped peripheral k-space segment (see orange k-space representation)
- Acquiring at least a dual echo gradient echo readout (balanced: also DESS readout)
- Use a Dixon reconstruction to separate water and fat
- From the separate water and fat reconstructions create a synthetic 4D CT (MRCAT) for radiation therapy planning

Four-dimensional respiratory resolved imaging can be achieved by a 3D radial stack of stars, Kooshball, 3D spiral stack of stars, or continuous sampling over a few minutes. Respiratory binning distinguishing inspiration and expiration states and outlier rejection is used for four-dimensional MR binning of the data.

Four-dimensional respiratory resolved imaging can be also applied to three-dimensional cartesian sampling with the so called 3D CASPR sampling that oversamples the three-dimensional central k-space compared to the peripheral k-space and covers k-space over a distributed sampling via spiral arms. Also three-dimensional spiral sampling may be an option.

A T2 preparation module with a three-dimensional gradient echo, preferably T2 - shifted echo - or balanced gradient echo readout is used. The shifted echo gradient echo sequence allows to create a large echo time needed for T2 gradient echo sampling, the balanced gradient echo balances the gradient readout achieving a T1/T2 contrast.

An inversion delay pulse or MTC pulse can be added to improve suppression of background (liver) signal and to enhance tumor visibility or for quantitative T1 mapping.

A dual or multiple echo gradient echo readout can be used. In case of the balanced sequence a DESS readout can be considered. The dual or multiple echo gradient echo can be used to reconstruct separate water and fat images using Dixon reconstruction. Those separate water and fat reconstructions can be used to create a synthetic four-dimensional CT (MRCAT) using artificial intelligence (AI) to identify water, fat and bone structures for radiation therapy planning.

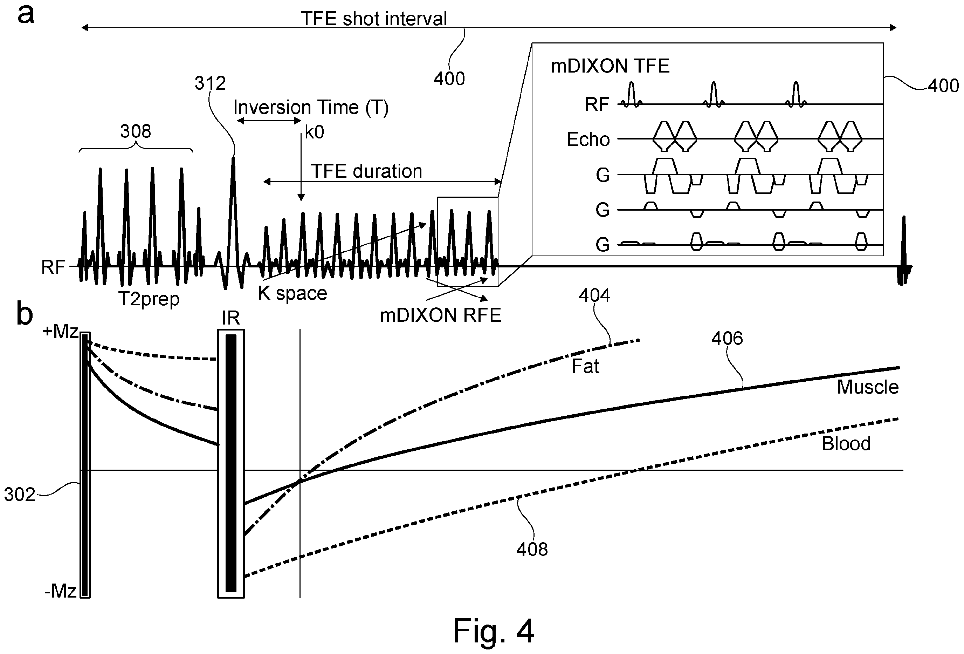

Fig. 4 illustrates a further example of a pulse sequence 400 for a single TFE shot. There is a T2 preparatory pulse 308 and an inversion recovery pulse 312 that can either precede the T2 preparatory pulse or not (as shown). After this, there is an m-Dixon TFE 402. The magnetization 302 for this TFE shot interval 400 is shown below. It can be seen that after the inversion recovery 312, the signals from the fat 404, the muscle 406, and the blood 408 are differentiated allowing to suppress the background signal efficiently by the optimal choice of the inversion time.

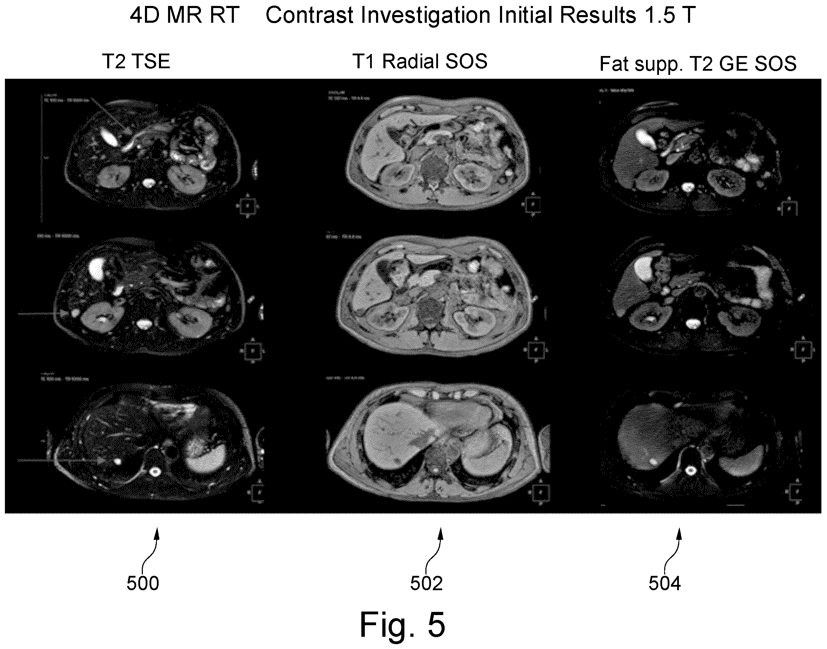

Fig. 5 shows three different slices in a magnetic resonance image using three different protocols. In these examples a T2 preparatory module before the balanced steady state free procession (bSSFP) gradient echo radial stack-of-stars was acquired with two echoes and reconstructed with a Dixon reconstruction. Column 500 uses a conventional T2 turbo spin echo pulse sequence. Column 2, 502, uses a T1 radial stack-of-stars. Column 504 uses a fat-suppressed T2 gradient echo with the stack-of-stars.

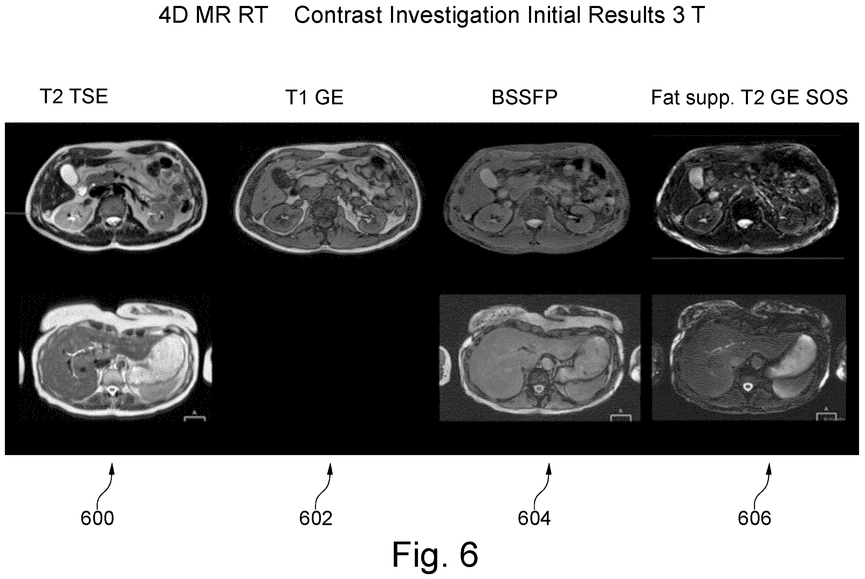

Fig. 6 illustrates further examples that compare three different magnetic resonance imaging protocols. Column 600 shows an image generated using standard T2 turbo spin echo pulse sequence. Column 602 shows an image reconstructed from the T1 gradient echo. Column 604 shows an image reconstructed using a balanced steady state free procession (bSSFP) sequence. Column 606 shows an image that used a fat-suppressed T2 prepared bSSFP gradient echo with a stack-of-stars pulse sequence.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: magnetic resonance imaging system
- 104: magnet
- 106: bore of magnet
- 108: imaging zone
- 109: field of view
- 110: magnetic field gradient coils
- 112: magnetic field gradient coil power supply
- 114: radio frequency coil
- 116: transceiver
- 118: subject
- 120: subject support
- 130: computer
- 132: computational system
- 134: hardware interface
- 136: user interface
- 138: memory
- 140: machine executable instructions
- 142: pulse sequence commands
- 144: k-space data
- 146: magnetic resonance image
- 200: repeatedly acquire the k-space data in the multiple shots by controlling the magnetic resonance imaging system with the pulse sequence commands
- 202: reconstruct at least one magnetic resonance image from the multiple shots of k-space data according to the magnetic resonance imaging protocol
- 300: portion of pulse sequence
- 302: magnetization
- 304: RF pulses
- 306: readout
- 308: T2-prepratory pulse
- 310: Turbo Field Echo readout
- 312: inversion pulse
- 314: T1 k-space data acquisitions
- 400: TFE shot interval
- 402: mDIXON TFE
- 404: fat signal
- 406: muscle signal
- 408: blood signal
- 500: T2 tubo spin echo image
- 502: T1 image with radial stack-of-stars sampling
- 504: fat-suppressed T2 gradient echo image with stack-of-stars sampling
- 600: T2 turbo spin echo image
- 602: T1 gradient echo image
- 604: bSSFP image
- 606: fat-suppressed bSSFP gradient echo image with stack-of-stars sampling

## Claims

1. A medical system (100) comprising:
- a magnetic resonance imaging system (102) configured for acquiring k-space data (144) from a subject (118) at least partially within an imaging zone (108);
- a memory (138) storing machine executable instructions (140) and pulse sequence commands (142) configured to acquire the k-space as multiple shots (400) of k-space data, wherein the pulse sequence commands are configured to cause the magnetic resonance imaging system to perform an initial spin echo preparatory pulse (308) with a refocusing flip angle of 180 degree or less, wherein the initial spin echo preparatory pulse is configured to prepare T2 or diffusion magnetization for each of the multiple shots, wherein the pulse sequence commands are further configured to acquire the k-space data using a Turbo Field Echo readout (310) with T1 weighting, balanced or T2 weighting, wherein the pulse sequence commands are configured to acquire the k-space data using a flip angle that decreases as a distance from a central k-space region increases; and
- a computational system (132), wherein execution of the machine executable instructions causes the computational system to:
- repeatedly (200) acquire the k-space data (144) in multiple shots by controlling the magnetic resonance imaging system with the pulse sequence commands, and
- reconstruct (202) at least one magnetic resonance image (146) from the multiple shots of k-space data according to the magnetic resonance imaging protocol.

2. The medical system of claim 1, wherein the pulse sequence commands are configured such that the k-space data is acquired using at least dual echo gradient echo readouts, wherein the at least one magnetic resonance image comprises any one of the following: a Dixon water image, a Dixon inphase image, a Dixon fat image, a Dixon outphase image, a Dixon B0 map, a Dixon T2* map, a Dixon Fat Fraction map, and combinations thereof.

3. The medical system of claim 2, wherein execution of the machine executable instructions further causes the computational system to reconstruct a synthetic computed tomography image using any one of the following: the Dixon water image, the Dixon fat image, the Dixon B0 map, the Dixon T2* map, the Dixon Fat Fraction map, and combinations thereof.

4. The medical system of claim 3, wherein execution of the machine executable instructions further causes the computational system to:
- receive a radiation therapy treatment plan identifying one or more regions of the subject to irradiate; and
- generate radiation therapy control commands configured to control a radiation therapy system to irradiate the one or more regions of the subject using the synthetic computed tomography image.

5. The medical system of any one of the preceding claims, wherein the pulse sequence commands are configured to acquire the multiple shots of k-space data with a golden angle sampling pattern varied by shot and by pulse sequence repetition, wherein the pulse sequence commands are further configured to preferably use any one of the following: a 3D radial stack of stars k-space sampling pattern, a 3D Kooshball k-space sampling pattern, a 3D spiral k-space sampling pattern, and a 3D cartesian trajectory with spiral profile k-space sampling pattern.

6. The medical system of any one of the preceding claims, wherein the pulse sequence commands are configured such that the multiple shots of k-space data are acquired using interleaved phase cycling.

7. The medical system of any one of the preceding claims, wherein the medical system further comprises a respiratory monitoring system configured for measuring respiratory data descriptive of a respiratory cycle of the subject, wherein the respiratory monitoring system is preferably any one of the following: an external k-space navigator, an intrinsic (self) k-space navigator, a FID navigator, an external sensor (3D camera), and combinations thereof; wherein execution of the machine executable instructions causes the computational system to repeatedly:
- acquire the multiple shots of k-space data by controlling the magnetic resonance imaging system with the pulse sequence commands;
- acquire the respiratory data using the respiratory monitoring system during the acquisition of the multiple shots of k-space data; and
- label the multiple shots of the k-space data using the respiratory data based on a time matching the respiratory data;
wherein execution of the machine executable instructions further causes the computational system to:
- bin the labeled multiple shots of the k-space data by the respiratory cycle, wherein the at least one magnetic resonance image is multiple magnetic resonance images for the respiratory cycle.

8. The medical system of claim 7, wherein the pulse sequence commands are configured such that the Turbo Field Echo readout has a T2 weighting, wherein the pulse sequence commands are configured such that the multiple shots occur at a varying delays after the initial spin echo preparatory pulse, wherein the machine executable instructions are configured such that the multiple shots of the k-space data are labeled using the respiratory data based on a time matching of the varying delays and the respiratory data, wherein the machine executable instructions are configured such that the labeled multiple shots of the k-space data are binned by the respiratory cycle and by the varying delays, wherein the machine executable instructions are configured such that the at least one magnetic resonance image is multiple magnetic resonance images for the respiratory cycle and for the respective varying delays, wherein execution of the machine executable instructions further causes the computational system to calculate a time dependent T2 relaxation time map from the at least one magnetic resonance image for the respiratory phase and for the varying delays.

9. The medical system of claim 8, wherein the pulse sequence commands are configured to cause the magnetic resonance imaging system to perform an inversion pulse after acquisition of the k-space data, wherein the pulse sequence commands are further configured to acquire T1 k-space data after the inversion pulse, wherein execution of the machine executable instructions causes the computational system to repeatedly:
- acquire the T1 k-space data in multiple shots by controlling the magnetic resonance imaging system with the pulse sequence commands, wherein the multiple shots occur at a varying times after the inversion pulse;
- acquire the respiratory data using the respiratory monitoring system during the acquisition of the T1 k-space data;
- label the multiple shots of the T1 k-space data using the respiratory data based on a time matching of the varying delays and the respiratory data;
wherein execution of the machine executable instructions further causes the computational system to:
- bin the labeled multiple shots of the T1 k-space data by the respiratory data and by the varying times after the invention pulse;
- reconstruct a T1 weighted image for the respiratory phase and for the respective varying times; and
- calculate a four-dimensional T1 relaxation time map from the T1 weighted image for the respiratory phase and for the varying times.

10. The medical system of claim 9, wherein execution of the machine executable instructions further causes the computational system to generate a four-dimensional synthetic magnetic resonance image from the four-dimensional T2 relaxation time map and the four-dimensional T1 relaxation time map.

11. The medical system of claim 10, wherein execution of the machine executable instructions further causes the computational system to:
- segment the four-dimensional synthetic magnetic resonance image using a segmentation algorithm to identify anomalous anatomical structures;
- overlay the segmentation of the anomalous anatomical structures on the four-dimensional synthetic magnetic resonance image.

12. The medical system of any one of the preceding claims, wherein the respiratory monitoring system is implemented using k-space navigators integrated into the pulse sequence commands, wherein execution of the machine executable instructions further causes the computational system to switch the k-space navigators between being leading navigators to trailing navigators.

13. A method of magnetic resonance imaging, wherein the method comprises:
- repeatedly (200) acquiring k-space data (144) in multiple shots (400) by controlling a magnetic resonance imaging system (102) with pulse sequence commands (142), wherein the pulse sequence commands are configured to cause the magnetic resonance imaging system to perform an initial spin echo preparatory pulse (308) with a refocusing flip angle of 180 degree or less, wherein the initial spin echo preparatory pulse (308) is configured to prepare T2 or diffusion magnetization for each of the multiple shots, wherein the pulse sequence commands are further configured to acquire the k-space data using a Turbo Field Echo readout (310) with T1 weighting, balanced or T2 weighting, wherein the pulse sequence commands are configured to acquire the k-space data using a flip angle that decreases as a distance from a central k-space region increases; and
- reconstructing (202) at least one magnetic resonance image from the multiple shots of k-space data according to the magnetic resonance imaging protocol.

14. A computer program comprising machine executable instructions configured for causing a computational system to perform the method of claim 13.

15. A computer-readable medium having stored thereon the computer program of claim 14
